Europäisches Patentamt

European Patent Office

Office européen des brevets

(11) Publication number: **0 410 696 A1**

# EUROPEAN PATENT APPLICATION

(21) Application number: **90308096.8**

(22) Date of filing: **24.07.90**

(51) Int. Cl.⁵: **A61K 9/70, A61K 9/00**

(30) Priority: **28.07.89 GB 8917317**
**19.12.89 US 452897**

(43) Date of publication of application:
**30.01.91 Bulletin 91/05**

(84) Designated Contracting States:
**AT BE CH DE DK ES FR GB GR IT LI LU NL SE**

(71) Applicant: **E.R. SQUIBB & SONS, INC.NC.**
**Lawrenceville-Princeton Road**
**Princeton New Jersey 08543-400(US)**

(72) Inventor: **Kellaway, Ian W.**
**9-Dan-y-Bryn Avenue**
**Radyr Cardiff(GB)**
Inventor: **Warren, Simon**
**229 Inverness Place**
**Roath Cardiff(GB)**
Inventor: **Timmins, Peter**
**5 Heathbank Avenue**
**Irby Wirral Merseyside(GB)**

(74) Representative: **Thomas, Roger Tamlyn et al**
**D. Young & Co. 10 Staple InnInn**
**London WC1V 7RD(GB)**

(54) Mucoadhesive hydrogels delivery system.

(57) A mucoadhesive hydrogel drug delivery system is provided which comprises polyacrylic acid cross-linked with between about 1 and 20 percent by weight of a polyhydroxy compound, and a therapeutically effective amount of the drug to be delivered.

EP 0 410 696 A1

## MUCOADHESIVE HYDROGELS DELIVERY SYSTEM

Oral delivery of numerous therapeutically useful drugs has been unsuccessful in many cases. For example, drug substances based on peptides or proteins may be degraded rapidly by gastric and intestinal enzymes responsible for the breakdown of dietary proteins. Other non-peptide, non-protein drugs may undergo extensive first-pass metabolism following absorption from the gastro-intestinal tract. Parenteral administration of such drugs may be less than desirable for several reasons, not the least of which is poor patient compliance.

R. Anders et al. in "Evaluation of Laminated Muco-Adhesive Patches for Buccal Drug Delivery" discuss absorption of drugs through mucosa, e.g. the buccal mucosa, directly into the bloodstream thereby avoiding gastric and intestinal enzymes, low pH of the stomach and first-pass metabolism as discussed above. Anders et al. discuss the use of laminated adhesive hydrogel patches composed of an impermeable backing layer and a mucoadhesive non-ionic hydrocolloid polymer containing the drug. The non-ionic polymer adhesives discussed by Anders et al. include hydroxyethylcellulose, hydroxy-propylcellulose, polyvinylpyrrolidone and polyvinylalcohol.

Park et al. in a paper in Pharm Res. , Vol. 4, No. 6, p. 457-464 (1987) disclose hydrogels for use on the gastric mucous layer. These adhesives comprise polyacrylic acid or polyacrylic acid-polyacrylamide copolymers, which were formed from acrylic acid and acrylamide, 2,5-dimethyl-1,5-hexadiene (DMDH) as a cross-linking agent and ammonium persulfate as an initiator of the polymerization. Park et al. teach that only between about 0.1 and 0.5 percent by weight of cross-linking agent should be used to maintain acceptable mucoadhesion. Park et al. disclose that loosely cross-linked polyacrylic acid has good adhesion to gastric mucosa but that an increase in crosslinker, i.e. beyond 0.5 percent by weight, provided a sharp drop in hydrogels ability.

In most cases, the more loosely a polymer such as polyacrylic acid is cross-linked, the greater the amount of swelling is expected to take place *in situ.* Cross linked polyacrylic acid hydrogels showing large degrees of swelling often show rapid drug release. Hence hydrogels with higher degrees of cross-linking enable better control of drug release, but according to prior art bioadhesion may be compromised.

In accordance with the present invention a novel hydrogel drug delivery system is provided which comprises polyacrylic acid cross-linked with between about 1 and 20 percent by weight of a polyhydroxy compound, and a therapeutically effective amount of the drug to be delivered.

Various other aspects of the invention are set forth in the claims and preferred features of the invention will now be described.

FIGURE 1 shows the equilibrium swelling ratios of various hydrogels of the present invention at pH 7.4.

FIGURE 2 shows a comparison of the rate of hydration with cure time for hydrogels containing 0.15 g sucrose/g polyacrylic acid.

FIGURE 3 shows the effects of crosslinker concentration and cure times on detachment force of examples of hydrogels according to the present invention from glycoprotein control.

FIGURE 4 shows a comparison of drug diffusion from hydrating hydrogel and buffer solution acorss a dialysis membrane.

The drug delivery system of the present invention is based on the polyacrylic acid polymer which is crosslinked with a polyhydroxy compound in an amount of between about 1 and 20 percent by weight of the adhesive composition to form a substantially insoluble hydrogel. This provides a much broader range of crosslinking densities for the ionic polyacrylic acid-based mucoadhesive systems while unexpectedly providing very good adhesion to the mucosa. This is indeed surprising since prior work with the polyacrylic acid systems, albeit in gastric mucosa, indicated that only a narrow range of crosslinking densities below 0.5 percent by weight provided acceptable adhesion. The finding of the present invention, that is, that between about 1 and about 20 percent by weight of a polyhydroxy crosslinker can be used, provides several useful advantages. First, the ability to substantially increase the crosslink density of the polyacrylic acid, i.e. by using the 1-20 percent by weight range of polyhydroxy crosslinking agent, provides for less swelling of the polymer itself. It is generally understood that the loosely crosslinked polymers can swell to dozens of times their original size and that drug release from such polymers is less than desirable. That is, it is faster than desirable and there is less control over the release rate. This excess swelling also means that the polymer density per unit area is reduced which may be responsible for lower adhesion. Also, the substantially broader range of cross-linking densities, as compared to the prior art, provides the potential for tailoring the present mucoadhesive system for desired release of a variety of drugs.

The present mucoadhesive hydrogels can be prepared from acrylic acid which must be polymerized or, in a preferred embodiment the system of the present invention utilizes linear polyacrylic acid which obviates the use of an initiator as needed in the prior art. Any linear polyacrylic acid is suitable for use in the present invention and those having molecular weight between 300,000 and $4 \times 10^6$ are preferred.

The polyhydroxy compounds suitable for use as the crosslinking agent in the present system can be any of the polyhydroxy compounds known for such purposes. Typically a carbohydrate such as sugars, cyclitols and the like are useful. Specific examples include, but are not limited to sucrose, sorbitol, mannitol, propylene glycol, glycerol and the like, with sucrose being most preferred.

The polyhydroxy compound is present within the adhesive composition, as mentioned above, in an amount of from about 1 to about 20 percent by weight of the composition, and preferably is present in an amount of from about 5 to about 20 percent by weight.

In a preferred embodiment the hydrogel drug release system is employed on the buccal mucosa, although it can be appreciated by those skilled in the art, given the broad range of possible crosslinking densities, that the present system should be adaptable to other environments.

The delivery system of the present invention can be used with any compatible drug and is suited for the release of peptide-based and protein-based drugs, though not limited to these.

The present system can be designed to provide a controlled release of these drugs over time and as is understood in the art, by varying the crosslinking density the release properties can be correspondingly modified.

Preferably, the hydrogel delivery system utilizes an occlusive or impermeable backing layer as are known and readily available in the art.

The mucoadhesive hydrogels of the present invention are prepared by known techniques. For example, a solution containing the desired proportions of acrylic acid or linear polyacrylic acid and polyhydroxy crosslinking compound can be poured into casting devices, degassed, dried to remove solvent and cured as required. Mucoadhesive hydrogels of the present invention are preferably cured at about 90°C for about 1-4 hours, with curing at or above 3 hours preferred. Mucoadhesive materials, cast and cured as above, can be cut into any desired shape, such as discs etc., and thereafter loaded with the drug by known methods.

It will thus be seen that one aspect of the invention provides a method of delivering a therapeutically effective amount of a drug through buccal mucosa comprising adhering to said mucosa an adhesive material capable of releasing a drug contained therein through said mucosa, wherein said adhesive containing the drug comprises between about 80 and 99 percent by weight of polyacrylic acid crossliked with between about 1 and 20 percent by weight of a polyhydroxy compound.

The present invention will be better understood from the following examples but is not limited to the details therein.

## EXAMPLE

### A. Preparation of Hydrogel Mucoadhesives

Several solutions comprising linear polyacrylic acid (Carbopol, B. F. Goodrich, m.w. 450,000) and sucrose (crosslinking agent) were prepared having varying amounts of sucrose ranging between 1 and 20 percent by weight of the solution. Alliquots of these solution were poured into casting devices, degassed and dried to remove solvent. The resulting films were cured at 90°C at times ranging between 1 and 4 hours. Cured, glassy hydrogel discs were cut to size (0.95 cm diameter) following hydration to the rubbery state under conditions of 97% relative humidity. The so-formed discs were then dried and stored desiccated until use in the following tests.

### B. Hydration Tests (Swelling)

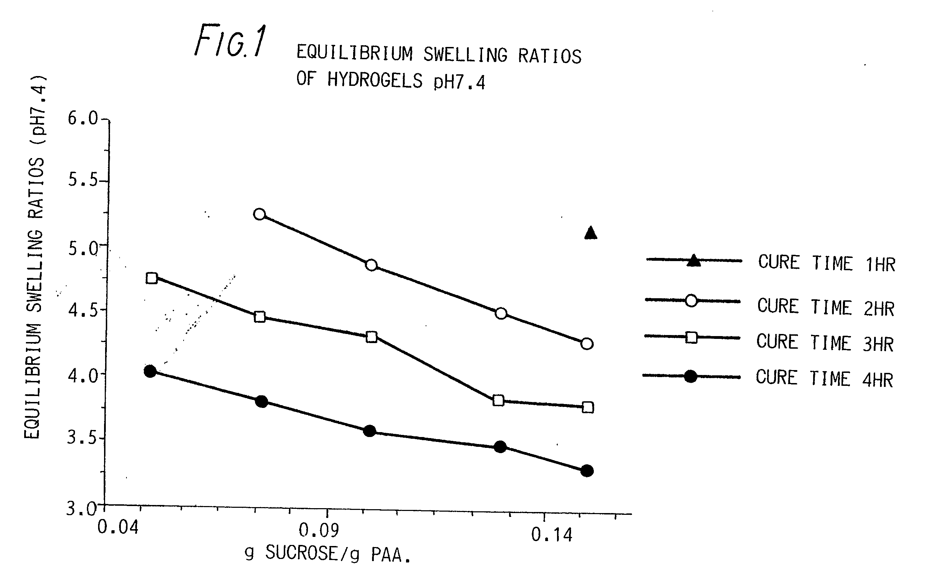

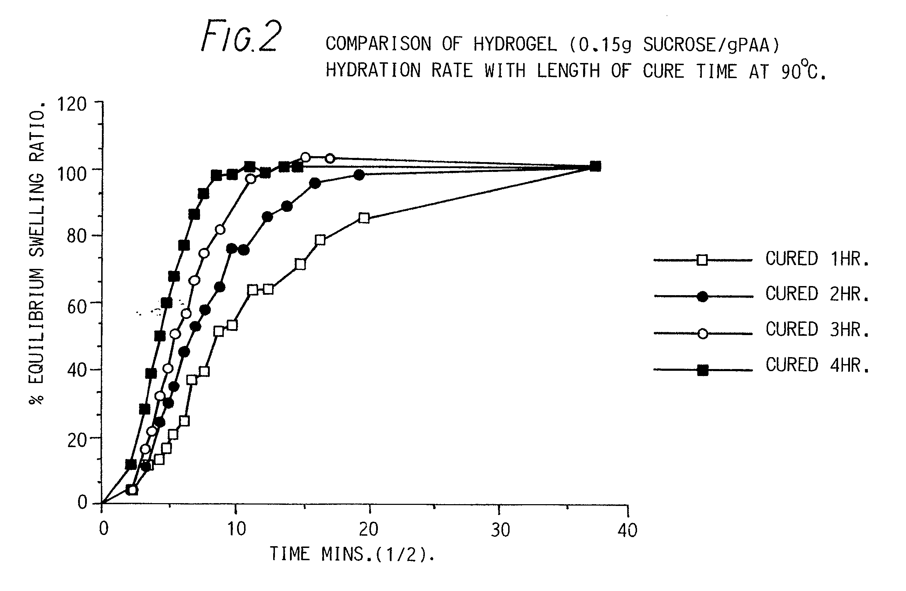

Hydrogel discs were varying amounts of crosslinker, as prepared above, were hydrated in a flat bottomed glass dish containing 100 ml of citrate/phosphate pH 7.4 buffer at about 22°C. The increase in diameter in discs caused by hydration (swelling) was measured at set time intervals with a linear scale placed beneath the glass dish. The results are illustrated in FIGURE 1 which generally shows the swelling decreasing with increasing crosslink density and/or cure time. FIGURE 2 illustrates the relationship of the rate of hydration versus the cure time for samples containing 0.15 g sucrose/g polyacrylic acid.

### C. Bioadhesion Tests

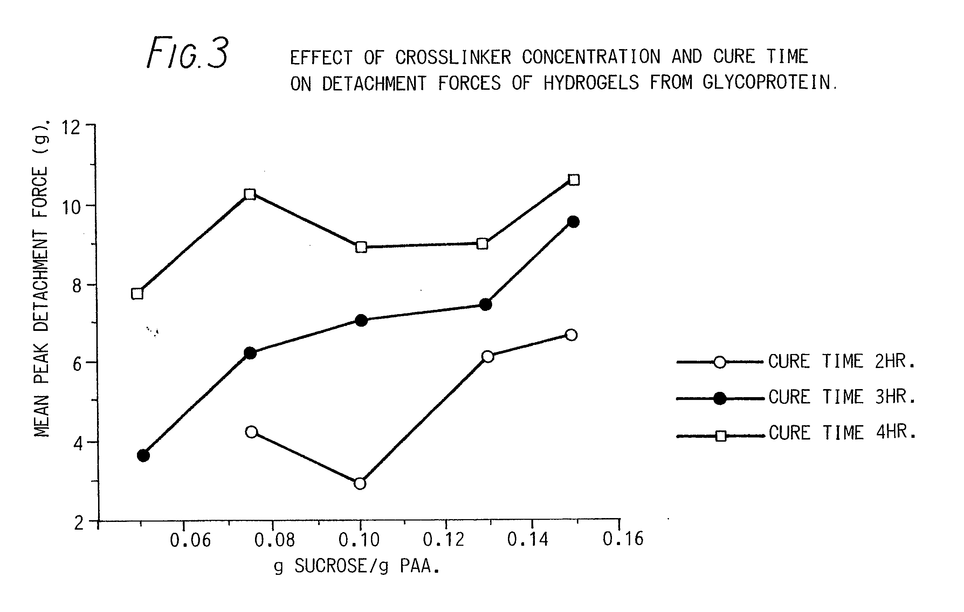

Samples of the mucoadhesive hydrogel discs as prepared in part A above, were fully hydrated in pH 7.4 citrate/phosphate buffer and the mean peak detachment forces from mucus glycoprotein were determined using the method developed by Kellaway et al., "Mucoadhesive Polymeric Systems", (Proc. 13th Int. Symp. Controlled Release of Bioactive Materials, 1986, p. 95). Adhesive materials were separated following a preload of 25 grams for 30 seconds at constant velocity (0.41 cm/min) from samples of freeze dried glycoprotein reconstituted (0.2 mg/ml) in citrate/phosphate pH 7.4 buffer. The results are illustrated in FIGURE 3 wherein the effects of crosslinker concentration and cure time on detachment forces are illustrated.

D. Drug Release Tests

Discs prepared as in part A (0.95 cm diam. by 0.045 cm thick) were loaded with a solution (25 μl per disc) containing 1 mg/ml of oxytocin acetate in distilled water in the following manner. Dry hydrogel discs were swollen to equilibrium in solutions of appropriate drug concentration. Hydrated discs were dried in a vacuum desiccator to constant weight. Drug loaded discs were then immersed in 2M hydrochloric acid for 10 minutes (i.e. non-swelling conditions) followed by successive washes in acetone to remove all traces of acid. Resultant films were vacuum dried to remove acetone.

E. Drug Release from Hydrating Gel

A two component glass cell separated by a dialysis membrane supporting the drug loaded hydrogel was securely clamped together. The recipient (lower) cell (5.5 ml vol) equipped with a side arm to allow sampling, was filled with citrate/phosphate buffer pH 7.4. The donor cell was sealed to ensure constant humidity. A cell containing 0.5 ml of a 0.05 mg/ml oxytocin solution in citrate/phosphate buffer pH 7.4 in the donor compartment, served as a control. Donor cells were stirred with magnetic bars and the complete apparatus placed in a water bath (22° C ± 2° C). 350μl samples were withdrawn from the recipient cells at set time intervals and replaced with an equal volume of fresh buffer. The samples were immediately derivatized with 200 μl of Fluorescamine (0.2 mg/ml in methanol). Quantification was achieved using an HPLC system employing a reversed phase CIS column and a fluorescence detector.

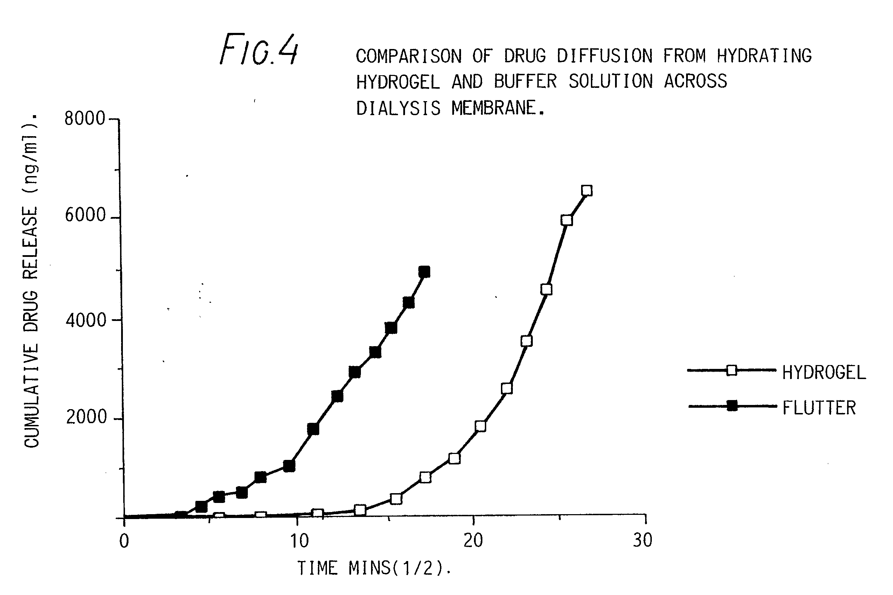

The lag time prior to steady state diffusion of an oxytocin solution through dialysis membrane (Fig. 4) was found to be 55 min. The peptide was first detected in the recipient solution after 20 min. In contrast peptide released from the drug loaded hydrogel was detected 120 mins. after the start of the experiment and steady state diffusion estimated at 352 min. The hydrogel used in the study was the most highly crosslinked and from the evidence of swelling studies the most hydrophilic.

Claims

1. A mucoadhesive delivery system comprising polyacrylic acid cross-linked with from about 1 to about 20 percent by weight of a polyhydroxy compound, and a therapeutically effective amount of a drug to be delivered.
2. The delivery system of claim 1 further comprising an impermeable backing layer.
3. The delivery system of claim 1 or 2 for use on the buccal mucosa.
4. The drug delivery system of claim 1, 2 or 3 wherein said polyacrylic acid is linear polyacrylic acid.
5. The drug delivery system of claim 1, 2, 3 or 4 wherein said polyhydroxy compound is selected from sugars, cyclitols, and lower polyhydric alcohols.
6. The drug delivery system of claim 5 wherein said polyhydroxy compound is selected from sucrose, galactose, sorbitol, mannitol, propylene glycol, glycerol, caradol and 1,3 propanediol.
7. The drug delivery system of any preceding claim wherein said polyhydroxy compound is present in an amount of from about 5 to about 20 percent by weight.
8. The delivery system of any preceding claim wherein said drug to be delivered is selected from peptide-based and protein-based drugs.
9. A buccal adhesive drug delivery system comprising a drug in an adhesive material capable of adhering to buccal mucosa and releasing said drug, and a backing layer laminated to a first surface of said adhesive opposite a second surface of said adhesive which contacts said mucosa;
wherein said adhesive comprises between about 80 and about 99 percent by weight of linear polyacrylic acid crosslinked with between about 1 and 20 percent by weight of sucrose.
10. A method of preparing a delivery system according to any one of claims 1-9, which comprises crosslinking polyacrylic acid with from about 1 to about 20 percent by weight of a polyhydroxy compound and loading the product with the drug.

FIG.1 EQUILIBRIUM SWELLING RATIOS
OF HYDROGELS pH7.4

EQUILIBRIUM SWELLING RATIOS (pH7.4)

g SUCROSE/g PAA.

CURE TIME 1HR
CURE TIME 2HR
CURE TIME 3HR
CURE TIME 4HR

EP 0 410 696 A1

FIG.2 COMPARISON OF HYDROGEL (0.15g SUCROSE/gPAA)
HYDRATION RATE WITH LENGTH OF CURE TIME AT 90°C.

CURED 1HR.
CURED 2HR.
CURED 3HR.
CURED 4HR.

EP 0 410 696 A1

FIG.3 EFFECT OF CROSSLINKER CONCENTRATION AND CURE TIME ON DETACHMENT FORCES OF HYDROGELS FROM GLYCOPROTEIN.

EP 0 410 696 A1

*FIG.4*    COMPARISON OF DRUG DIFFUSION FROM HYDRATING HYDROGEL AND BUFFER SOLUTION ACROSS DIALYSIS MEMBRANE.

## DOCUMENTS CONSIDERED TO BE RELEVANT

EP 90308096.8

| Category | Citation of document with indication, where appropriate, of relevant passages | Relevant to claim | CLASSIFICATION OF THE APPLICATION (Int. Cl.5) |
|---|---|---|---|
| X | EP - A1 - 0 275 550 (TEIKOKU SEIYAKU) * Page 2, line 35 - page 5, line 10 * | 1-4, 8-10 | A 61 K 9/70 A 61 K 9/00 |
| X | US - A - 3 220 960 (O. WICHTERLE et al.) * Columns 1,2; claims * | 1,3-6, 10 | |
| A | PATENT ABSTRACTS OF JAPAN, unexamined applications, C field, vol. 6, no. 57, April 14, 1982 THE PATENT OFFICE JAPANESE GOVERNMENT page 21 C 98 * Kokai-no. 56-169 623 (NITTO DENKI KOGYO K.K.) * | 1,2 | |

TECHNICAL FIELDS
SEARCHED (Int. Cl.5)

A 61 K 9/00

The present search report has been drawn up for all claims

| Place of search | Date of completion of the search | Examiner |
|---|---|---|
| VIENNA | 12-11-1990 | IRMLER |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)